# EUROPÄISCHE PATENTSCHRIFT

(11) **EP 0 279 358 B1**
(45) Veröffentlichungstag und Bekanntmachung des Hinweises auf die Patenterteilung: **29.06.1994**
(21) Anmeldenummer: 88102014.3
(22) Anmeldetag: 11.02.1988
(51) Int. Cl.: A61B 10/00, A61B 17/32

(54) **Medizinische Vorrichtung**
Medical device
Dispositif médical

(30) Priorität: 18.02.1987 DE 8702466 U
(43) Veröffentlichungstag der Anmeldung: 24.08.1988
(73) Patentinhaber: Kothe, Lutz, D-78315 Radolfzell (DE)
(72) Erfinder: Kothe, Lutz, D-78315 Radolfzell (DE)
(74) Vertreter: Weiss, Peter, Dr. rer.nat.

(56) Entgegenhaltungen:
- EP-A- 0 065 054
- EP-A- 0 119 405
- DE-U- 8 715 139
- FR-A- 2 479 680
- GB-A- 2 161 707
- US-A- 4 603 694
- US-A- 4 685 472

## Beschreibung

Die Erfindung betrifft eine medizinische Vorrichtung mit einer Schneidzangeneinrichtung zum Entnehmen von Gewebeproben, Entfernen von langgestreckten Körperelementen, wie Nerven- oder Venenabschnitten od. dgl., wobei ein Innenrohr in ein Außenrohr eingesetzt und ein Rohr axial gegen das andere Rohr verschiebbar ist und wobei ein Rohr die Schneidzangeneinrichtung besitzt, deren Schließbewegung von dem jeweils anderen Rohr abhängig ist, wobei die Schneidzangeneinrichtung aus zwei Schneidblättern mit Schneiden besteht, welche sich von jeweils einem gemeinsamen Drehpunkt mit einem Rohr zu einem gegenüberliegenden gemeinsamen Drehpunkt mit dem Rohr erstrecken.

Bei der Entnahme von Gewebeproben werden sogenannte Probeexzisionszangen verwendet, welche in den menschlichen Körper eingeführt werden, eine Gewebeprobe abklemmen oder abschneiden und beim Herausziehen dieser Zange halten. Sodann wird die Gewebeprobe auf einer entsprechenden Unterlage abgelegt.

Sollen mehrere Gewebeproben entnommen werden, bedeutet dies, daß für jede einzelne Probe die Probenexzisionszange erneut in den menschlichen Körper eingeführt werden muß, wobei es infolge von Gewebeverschiebungen häufig schwierig ist, wieder die gewünschte Stelle zu finden, von der ncoh keine Probe entnommen worden ist.

Bei den bekannten Probenexzisionszangen wird außerdem die Schneidbewegung der Schneidzangeneinrichtung in der Regel von einem in der Seele eines Rohres geführten Innenstab bewirkt, was es unmöglich macht, daß etwa durch diese Seele die abgeschnittene Probe entnommen werden kann.

Aus eben letztgenanntem Grund sind derartige Vorrichtungen auch nur zur Entnahme von Gewebeproben verwendbar. Werden sie beispielsweise beim Entfernen von langgestreckten Körperelementen, wie Nerven- oder Venenabschnitten, verwendet, so müssen an den entsprechenden Körperteilen in Abstand zueinander Operationsschnitte gelegt werden und so das zu entfernende Körperelement Stück für Stück abgeschnitten werden.

Aus der EP-A 65 054 ist eine Biopsizange bzw. -pinzette bekannt, bei welcher in einem Außenrohr ein Innenrohr gleitet. Stirnwärtig geht das Innenrohr über entsprechende Federstreifen in zwei Zangenschenkel über, welche elastisch, federnd mit dem Innenrohr verbunden sind. Endwärtig sind die Zangenschenkel nach außen aufgebogen, so daß sie beim Einfahren des Innenrohres durch die stirnwärtige Kante des Außenrohres zueinander bewegt werden und eine Klemmstellung einnehmen können. Damit das Innenrohr im Verhältnis zum Außenrohr nicht verdreht werden kann, sind in das Außenrohr stirnwärtig offene Senken eingeformt, welche die Zangenschenkel seitlich umfassen. Durch eine derartige Biopsiezange kann aber nur eine Klemmbewegung und keine Schneidbewegung vollzogen werden, da sich die Klemmschenkel nicht scherend zueinander bewegen können.

Gleiches gilt auch für eine Biopsiezange bzw. -pinzette wie sie in der FR-A 2 479 680 gezeigt ist. Dort besitzen zwar die beiden Zangenschenkel gemeinsame Drehpunkte, allerdings wird keine Schneidbewegung durchgeführt.

Aus der Europäischen Patentanmeldung 0 119 405 ist beispielsweise ein chirurgisches Instrument bekannt, bei dem zwei Rohre ineinander verschiebbar geführt sind, wobei das Innenrohr die Schließbewegung eines Schneidblattes vollführt. Dieses Instrument hat zum einen den Nachteil, daß seine Öffnungsweite sehr beschränkt ist. Ferner ist seine Schließbewegung, d.h., auch Schneidwirkung allein von dem Druck abhängig, unter dem das innere Rohr geführt ist.

Zur Entnahme von Gewebeproben von beispielsweise glatten Gewebewänden, auf welche dieses Instrument etwa rechtwinklig auftrifft, ist es nicht geeignet.

Die FR-A 2 479 680 zeigt eine Zange, bei welcher zwei Zangenbacken durch den Druck eines inneren Rohres gespreizt und durch den Zug geschlossen werden. Auch hierbei läßt die Kraftübertragung sehr zu wünschen übrig. Ein Abschneiden von Gewebeproben oder ein Entfernen von langgestreckten Körperelementen ist durch dieses Instrument nicht möglich.

Der Erfinder hat sich zum Ziel gesetzt, eine Vorrichtung der o.g. Art zu entwickeln, mittels welcher es möglich ist, eine Vielzahl von Gewebeproben zu entnehmen, ohne daß die Vorrichtung aus dem menschlichen Körper herausgezogen werden muß. Ferner soll diese Vorrichtung nicht nur zur Entnahme von Gewebeproben anwendbar sein, sondern auch für andere operative Maßnahmen, wie beispielsweise dem Entfernen von langgestreckten Körperelementen, wie Nerven- oder Venenabschnitte. Ferner soll die Kraftübertragung auf die Schneidzangeneinrichtung wesentlich verbessert werden, so daß es nicht mehr zu einem Abquetschen von beispielsweise Gewebeproben oder den langgestreckten Körperelementen, sondern zu einem glatten Abschneiden kommt.

Zur Lösung dieser Aufgabe führt, daß das jeweils andere Rohr stirnwärtig offene Senken in der Stirnkante aufweist, in welche beim Schließen der Schneidzangeneinrichtung durch das axiale Verschieben der Rohre die Drehpunkte einfahren, wobei Ränder der Senken an den Schneiden gegenüberliegenden Außenrändern der Schneidblätter entlanggleiten und die Schneidzangeneinrichtung schließen.

Bei einer derartigen Vorrichtung ist für die Schließbewegung der Schneidzangeneinrichtung kein Seilzug mehr notwendig, so daß die Seele des Innenrohres zum Entnehmen von Gewebeproben verwendet werden kann. Dieses Entnehmen geschieht der Einfachheit halber mittels eines Vakuums, wie dies weiter unten beschrieben ist.

Als geeignete Schneidzangeneinrichtung haben sich zwei Schneidblätter erwiesen, welche Schneiden und einen gemeinsamen Drehpunkt mit dem Rohr aufweisen. Dabei können die Schneidblätter entweder am Innenrohr außen oder am Außenrohr innen befestigt sein. Bevorzugt wird die Befestigung am Innenrohr außen.

Das jeweils andere Rohr, welches keine Schneidzangeneinrichtung trägt, weist Senken in der Stirnkante auf, in welche beim Schließen der Schneidzangeneinrichtung die Drehpunkte einfahren können Dabei treffen zuerst die Senkenränder auf die Aussenränder der Schneidblatter, heben diese an und umschließen sie in Schließlage. Zu diesem Zweck sind die Ränder der Senken den Außenrändern der Schneidblätter angepaßt. Dies wird bevorzugt, ist aber nicht zwingend.

Auf diese äußerst einfache Maßnahme ist ein sicheres Schließen der Schneidblätter zurückzuführen, wobei die abgeschnittene Gewebeprobe sofort abgesaugt wird. Die Vorrichtung braucht nicht aus dem menschlichen Körper herausgezogen werden, sondern kann in der Nähe der gerade entnommenen Gewebeprobe weitere Proben schneiden.

In der Praxis können Schwierigkeiten insofern auftauchen, als sich die Schneidblätter nach dem Abschneiden einer Gewebeprobe nicht selbsttätig öffnen. Hier können entsprechende Federelemente Abhilfe schaffen, was aber wiederum mit erheblichem konstruktiven Aufwand verbunden ist. Der Einfachheit halber wird deshalb ein mit den Schneidblättern in Eingriff stehender Bolzen vorgesehen, welcher beim Zurückführen des die Schneidbewegung verursachenden Rohres die Schneidblätter mitnimmt und öffnet. Bevorzugt wird hier eine in dem Schneidblatt ausgebildete Führungsausnehmung, welche als Kulisse für den Bolzen wirkt. Jedoch soll hier dem erfinderischen Gedanken keine Grenze gesetzt sein.

In einem anderen Ausführungsbeispiel der Erfindung ist vorgesehen, daß die Schneidblätter über Befestigungslaschen mit dem Rohr verbunden sind. Dabei besitzen diese Befestigungslaschen einen Haken, mit dem sie in eine entsprechende Ausnehmung in den Schneidblättern eingreifen, während sie andernends über Bolzenstücke od. dgl. mit dem Rohr verbunden sind. Diese Bolzenstücke sind dann der Einfachheit halber in das Rohr eingeschweißt. Die Befestigungslaschen haben dabei insbesondere die Funktion, die Schneideinrichtung zu öffnen. Das Schließen geschieht auch hier über entsprechende Senken, welche in die Stirnkante des Rohres eingeformt sind.

In einem einfachen Ausführungsbeispiel der Erfindung ist vorgesehen, daß die Schneidblätter Eingriffsmulden aufweisen, in welche entsprechende Rastnasen des Rohres eingreifen.

Beim Öffnen und Schließen rollen diese Rastnasen quasi in den Eingriffsmulden ab, wobei daneben eine entsprechende Eingriffsmulde im Rohr gebildet ist, in welche wiederum entsprechende Rastnasen von den Schneidblättern aus eingreifen. Auf diese Weise wird ein Rollenlager gebildet.

Soll die erfindungsgemäße Vorrichtung nicht zur Entnahme von Gewebeproben dienen, sondern beispielsweise zum Entfernen von langgestreckten Körperelementen, so ist selbstverständlich auch kein Vakuumanschluß notwendig. In diesem Fall besitzen sowohl Außen- wie auch Innenrohr einen Kerbschnitt, welche den Zugriff zur Seele des Innenrohres ermöglichen. Auf diese Weise kann beispielsweise eine Nervenbahn in die Mündungsöffnung des Innenrohres eingeführt werden und zu den Kerbschnitten herauslaufen. Der Operateur kann das Nervenende halten, während er die Vorrichtung weiter über die Nervenbahn schiebt und an einem entfernten Ende abschneidet.

Zu dem eben genannten Zweck kann es auch notwendig sein, sowohl Außen- wie auch Innenrohr aus flexiblem Material herzustellen.

Bei dieser einfachen Ausführungsform der erfindungsgemäßen Vorrichtung genügt als Betätigungseinrichtung, daß das Innenrohr fest mit einem Griffstück verbunden ist, während sich das Außenrohr gegen einen axial bewegbaren Schraubkopf abstützt. Beim Druck auf den Schraubkopf wird das Außenrohr axial verschoben und schließt die Schneidzangeneinrichtung. Zum Öffnen wird das Außenrohr einfach durch Zurückziehen des Schraubkopfes ebenfalls axial verschoben.

Es kann sich in manchen Fällen auch als günstig erweisen, wenn der Schraubkopf in diesem Fall mit einem Gewindeabschnitt in das Innenrohr eingreift, so daß seine Kraft auf das Außenrohr durch Schrauben übertragen wird. Sobald ein bestimmter Anschlag erreicht ist, weiß der Operateur, daß die Schneidzangeneinrichtung geschlossen ist. Auch die Kraftübertragung kann durch die Schraubbewegung verbessert sein. In diesem Fall wird es sich als günstig erweisen, wenn das Außenrohr geführt ist, was der Einfachheit halber durch einen Stift in dem Griffstück geschieht, welcher ein Langloch in dem Außenrohr durchsetzt.

Dies kann sich aber auch tei der Verwendung als Entnahmezange als nützlich erweisen und zwar beispielsweise beim Entfernen von Gallensteinen od. dgl.. Kleine Steine können durch die Schneidzangeneinrichtung selbst zertrümmert und die Trümmerbrocken durch die Seele des Innenrohres abgesaugt werden. Dasselbe gilt auch, wenn ein Zertrümmern von Steinen durch bekannte Vorrichtungen vor der Entnahme der Brocken stattgefunden hat.

In dem bevorzugten Ausführungsbeispiel, bei dem das Innenrohr die Schneidzangeneinrichtung trägt und das Außenrohr zum Schließen dieser Einrichtung dient, soll das Außenrohr über Gelenklaschen mit einer dem Zangenschenkel angeformten Hülse verbunden sein, welche das Innenrohr spielfrei umgibt. Hierdurch wird eine entsprechend notwendige Beweglichkeit der Verbindung gewährleistet.

Ferner durchsetzt das Innenrohr eine dem anderen Schenkel angeformte Hülse und weist andererseits einen Anschlußnippel auf. Auf diesen Anschlußnippel kann eine Leitung aufgesteckt werden, welche zu einer Vakuumpumpe führt.

Eine andere Ausführungsform der Betätigungsvorrichtung sieht vor, daß das Außenrohr fest mit einem Gehäuse verbunden ist, in welchem sich eine Ventileinrichtung befindet, die eine Verbindung zwischen einer Axialbohrung im Innenrohr und der Leitung zur Vakuumpumpe herstellt oder unterbricht. In der Praxis hat es sich als ratsam erwiesen, daß die medizinische Einrichtung nicht die gesamte Arbeitszeit unter Vakuum gesetzt ist. Wird beispielsweise die Vorrichtung im menschlichen Körper bewegt, so kann es über eine ganze Strecke hinweg notwendig werden, daß nicht Fremdmaterial aufgesaugt wird.

Diese Ventileinrichtung kann so aussehen, daß das Innenrohr mit einem Einsatz verbunden ist, welcher andererseits mit einem Rohrstück in den Anschlußnippel eingreift. Dieser Einsatz ist verschiebbar in einer Mulde des Gehäuses gelagert. In dem Einsatz selbst ist ein Drehkopf drehbar angeordnet, welcher eine Querbohrung zum Verbinden von Axialbohrung und Leitung besitzt. Dieser Drehkopf greift mit einem Lagerstift in das Gehäuse ein und ist andererseits an einen Zangenschenkel angeformt, welcher eine entsprechende Ausnehmung im Gehäuse durchsetzt. Wird dieser Zangenschenkel bewegt, so verschiebt er den Einsatz, wobei gleichzeitig der Drehkopf relativ zum Einsatz gedreht wird und die Querbohrung aus der Axialflucht mit der Axialbohrung und der Leitung gerät. Hierdurch wird das Ventil geschlossen.

Zum Auffangen der Gewebeproben ist in die Leitung eine Auffangvorrichtung eingeschaltet. Diese sieht zumindest ein Auffangvlies vor. Auch hier sind jedoch kompliziertere Einrichtungen denkbar, wobei die Auffangvorrichtung so ausgestaltet ist, daß sie nach dem Auffangen einer Gewebeprobe automatisch öffnet und ein nächstes Auffangvlies eingesetzt wird.

Der Vakuumanschluß hat noch einen weiteren erheblichen Vorteil bei der Entnahme von Gewebeproben. Beim Aufsetzen des Kopfbereiches der Vorrichtung auf das Gewebe wird dieses teilweise in die Mündungsöffnung des Innenrohres eingesaugt, so daß eine größere Gewebeprobe entnommen werden kann. Insbesondere bei der Gewebeprobeentnahme von glatten Flächen hat sich dies als besonders wirkungsvoll herausgestellt. Ferner sind die Schneidblätter so angeordnet, daß sie vorausschneidend sind, d. h. immer die Mündungsöffnung des Innenrohres übergreifen.

Diese neue entwickelte Tumor- und Probenexzisionszange ist insbesondere für folgende Anwendungsbereiche gedacht:
- Biopsie aus soliden Geweben, Tumoren, Lymphknoten und Wänden der Hohlorgane;
- endoskopische Abtragung oberflächlicher Veränderungen wie Polypen, Tumore, Granulome usw.;
- endoskopische Biopsie aus Oberflächenveränderungen wie Ulcera, Entzündungen, Plaques und sonstigen Schleimhautveränderungen;
- Entfernung freier Gelenkkörper und degenerativer Materialien aus großen Gelenken.

Die erfindungsgemäße Vorrichtung soll in der Gynäkologie bei endoskopischen Eingriffen an Vagina, Portio und Uterus, in der Gastroenterologie bei der Endoskopie der unteren Dickdarmabschnitte, in der Pulmologie bei der Mediastinoskopie und starren Bronchoskopie sowie bei der Mini-Thorakotomie, in der HNO-Heilkunde bei der Laryngoskopie und bei der Endoskopie der Nasennebenhöhlen, in der Urologie bei der Cystoskopie und evtl. Prostatachirurgie und in der Orthopädie bei der Arthroskopie großer Gelenke Anwendung finden.

Bei einer technischen Weiterentwicklung zum flexiblen Instrument wäre der Einsatz im Rahmen der Ösophago-Gastro-Duodenoskopie, der Coloskopie, der tiefen Bronchoskopie und der Arthroskopie mittlerer Gelenke denkbar.

Die besonderen Vorteile sind:
- Durch quetschungsfreien Schnitt werden die Gewebeproben geschont, die histologische Auswertung wird erleichtert und sicherer.
- Der besondere Schneidemechanismus erlaubt eine Probeentnahme, ohne daß das Instrument gegen das Gewebe gedrückt werden muß, wie dies bei herkömmlichen Zangen der Fall ist. Hierdurch ergibt sich eine definierte Schnittiefe und ein definiertes Volumen des entnommenen Gewebes. Die Gefahr der Perforation wird dadurch minimiert, die Anzahl der Entnahmeversuche durch das ausreichend große Gewebestück wird verringert.
- Die direkte Absaugung der Gewebeteilchen erlaubt es, dem Untersucher die Biopsiestelle ständig mit der Optik im Auge zu behalten. Instrument und Optik können so stets auf das interessierende Areal gerichtet bleiben, während bei der bisher üblichen Probeentnahme das Gewebe mit der Zange selbst entfernt werden mußte. Dies bedeutet ein ständiges Ein- und Ausführen der Zange. Ein Untersuchungsgebiet muß also nicht mehrmals neu mit der Biopsiezange aufgesucht werden.
- Ein zügiges, rationelles und für den Patienten schonenderes Untersuchungsverfahren ist gegeben. Auch die Abtragung größerer Gewebeteile, wie beispielsweise breitbasig aufsitzender Tumore, wird damit möglich gemacht.
- Die Sicherheit bioptischer Untersuchungen ist vergrößert, für den Patienten kann daraus eine strengere Indikationsstellung für invasivere Behandlungen, wie Operationen, resultieren.
- Komplikationen bei der Biopsie, wie beispielsweise Nachblutungen o. ä., können rasch erkannt und angegangen werden, weil der Blick des Untersuchers auf die Stelle der Probeentnahme gerichtet bleibt.
- Durch die Biopsiezange ist ein Absaugen von Flüssigkeiten ebenfalls möglich. Einerseits kann damit ein gutes Sichtverhältnis bei der Endoskopie geschaffen werden, beispielsweise durch Absaugen von Blut oder Sekreten, ohne daß ein Wechsel des Arbeitsinstruments erforderlich wird.

Andererseits ist mit der Biopsiezange selbst die Entnahme von Flüssigkeiten zu diagnostischen Zwecken ermöglicht, zum Beispiel als Spülcytologie.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
- Fig. 1: eine teilweise geschnittene, perspektivische Darstellung einer erfindungsgemäßen Probenexzisionszange in Gebrauchslage;
- Fig. 2: eine Draufsicht auf ein vergrößert dargestelltes Ausführungsbeispiel eines Schneidbereichs einer Zange nach Fig. 1 in Schließlage;
- Fig. 3: eine Draufsicht auf das Ausführungsbeispiel nach Fig. 2 in geöffneter Lage;
- Fig. 4: eine perspektivische Ansicht eines Außenrohres eines weiteren Ausführungsbeispiels der erfindungsgemäßen Zange;
- Fig. 5: eine perspektivische Draufsicht auf das zu Fig. 4 gehörende Innenrohr mit Schneidelementen;
- Fig. 6: eine perspektivische und teilweise gebrochen dargestellte Ansicht eines weiteren Ausführungsbeispiels eines Schneidbereiches einer erfindungsgemäßen Zange;
- Fig. 7: eine Draufsicht auf ein Schneidblatt, welches in dem Ausführungsbeispiel gemäß Fig. 6 Anwendung findet;
- Fig. 8: eine perspektivische Ansicht eines weiteren Ausführungsbeispiels eines Schneidbereichs in Schließlage;
- Fig. 9: eine perspektivische Ansicht des Schneidbereichs nach Fig. 8 in Öffnungslage;
- Fig. 10: einen Längsschnitt durch ein weiteres Ausführungsbeispiel einer Probenexzisionszange im Bereich des Griffs mit geöffnetem Ventil;
- Fig. 11: Den Längsschnitt nach Fig. 10 mit geschlossenem Ventil;
- Fig. 12: eine Draufsicht auf ein weiteres Ausführungsbeispiel einer Probenexzisionszange;
- Fig. 13: eine teilweise geschnitten dargestellte Seitenansicht der Zange nach Fig. 12.

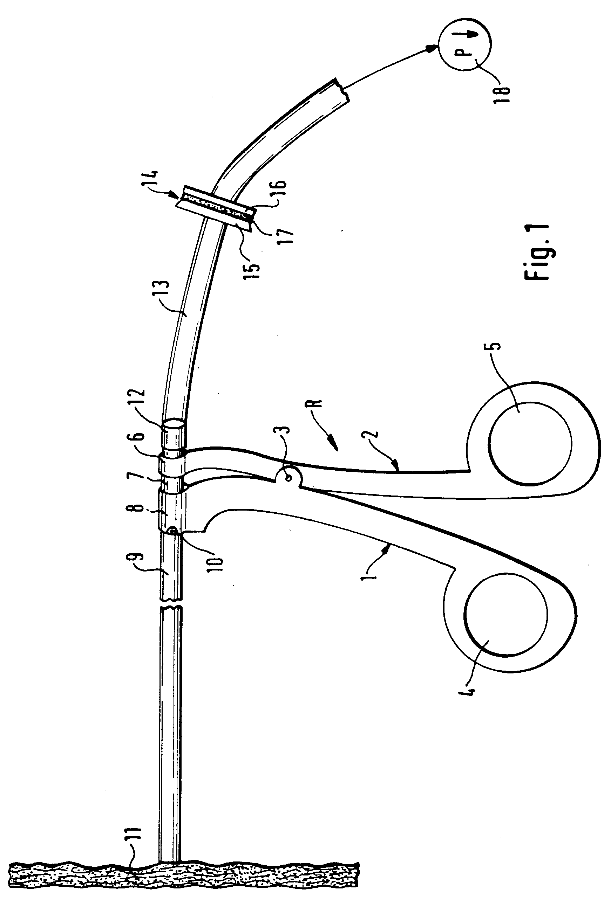

Eine Probenexzisionszange R weist gemäß Fig. 1 zwei Zangenschenkel 1 und 2 auf, welche über ein Gelenk 3 miteinander verbunden sind. Einerseits besitzt jeder Zangenschenkel 1 bzw. 2 eine Grifföffnung 4 bzw. 5, in welche jeweils ein Finger einer menschlichen Hand einführbar ist. Andererseits des Gelenks ist dem Zangenschenkel 2 eine Hülse 6 angeformt, in welche ein Innenrohr 7 eingesetzt ist.

Auch der Zangenschenkel 1 besitzt andererseits des Gelenkes eine Hülse 8, welche das Innenrohr 7 spielfrei umfängt und mit einem Außenrohr 9 über Gelenklaschen 10 verbunden ist.

Das Außenrohr 9 umgibt das Innenrohr 7 und trifft in Gebrauchslage zusammen mit diesem auf einen Gewebeabschnitt 11 im menschlichen Körper.

Das Innenrohr 7 durchragt die Hülse 6 andererseits der Hülse 8, wobei es einen Anschlußnippel 12 für eine Schlauchleitung 13 bildet. In diese Schlauchleitung 13 ist eine Auffangvorrichtung für Gewebeproben eingeschaltet, welche beispielsweise aus zwei miteinander gekoppelten Lochscheiben 15 und 16 besteht, zwischen welche ein Auffangvlies 17 eingelegt ist. Nach dieser Auffangvorrichtung 14 führt die Leitung 13 zu einer nur schematisch angedeuteten Vakuumpumpe 18.

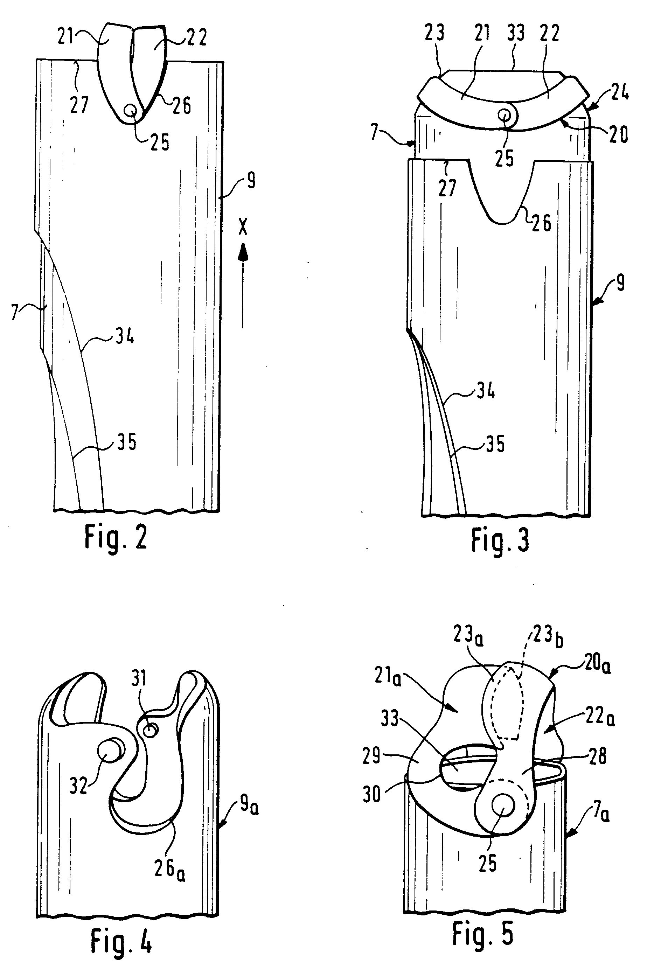

In den Figuren 2 bis 9 sind verschiedene Ausführungsformen von Schneidbereichen der Probenexzisionszange dargestellt, welche in Gebrauchslage auf den Gewebeabschnitt 11 nach Fig. 1 treffen. Erfindungsgemäß ist im Kopfbereich eine Schneidzangeneinrichtung 20 vorgesehen, welche aus zwei Schneidblättern 21 und 22 besteht. Jedes Schneidblatt besitzt eine Schneide 23. Beide Schneidblätter 21 und 22 umfangen in geöffneter Lage den abgerundeten Kopfbereich 24 des Innenrohres 7, wobei sie gemeinsame, sich gegenüberliegende Drehpunkte 25 besitzen, mittels denen sie auch am Innenrohr 7 festgelegt sind.

Das Außenrohr 9 weist in seinem Scheitelbereich eine Senke 26 auf, deren Innenkontur in etwa der Außenkontur der Schneidblätter 21 bzw. 22 angepaßt ist.

Zum Schließen der Zange wird das Außenrohr 9 gemäß Fig. 2 in Richtung x verschoben, wobei es mit seiner Stirnkante 27 auf die Schneidblätter 21 und 22 trifft. Diese werden aufgestellt, wobei der Drehpunkt 25 in die Senke 26 einfährt. In Schließlage treffen die beiden Schneiden 23 der Schneidblätter 21 und 22 aufeinander und können so eine Gewebeprobe abschneiden. Diese Gewebeprobe wird durch das an das Innenrohr 7 angelegte Vakuum abgesaugt und im Auffangvlies 17 aufgefangen.

Zum verdrehsicheren Lagern von Innenrohr 7 zu Außenrohr 9 können beiden Rohren entsprechende, nicht näher gezeigte Zentriernute bzw. -vorsprünge eingeformt sein.

In den Figuren 4 und 5 ist ein weiteres Ausführungsbeispiel einer Schneidzangeneinrichtung 20a dargestellt. Auch hier besteht die Schneidzangeneinrichtung 20a aus zwei Schneidblättern 21a und 22a, welche einen gemeinsamen Drehpunkt 25 mit dem Innenrohr 7a bilden. Bei dieser Schneidzangeneinrichtung 20a sind die Schneidblätter 21a und 22a so ausgebildet, daß die Schneide 23a des einen Schneidblattes 22a die Schneide 23b (gestrichelt dargestellt) des anderen Schneidblattes 21a überlappt.

Jedes Schneidblatt 21a bzw. 22a besitzt einerseits eine Zunge 28 zum einen Drehpunkt 25 hin und zum anderen Drehpunkt 25 hin eine Schwinge 29, welche eine Führungsausnehmung 30 ausbildet.

Wird dieses Innenrohr 7a in das Außenrohr 9a gemäß Fig. 4 eingesetzt, so greifen Bolzen 31 in die jeweilige Führungsausnehmung 30 ein. Der Bolzen 31 kann beispielsweise in das Material des Außenrohres 9a eingedreht sein, weswegen von außen noch ein Bolzenkopf 32 sichtbar ist.

Zum Schließen der Schneidzangeneinrichtung 20a fährt die Zunge 28 des Schneidblattes 22a in die Senke 26a ein, wobei das Schneidblatt 22a aufgestellt wird. Auf der anderen Seite fährt ebenfalls die Zunge des Schneidblattes 21a in die ihr zugeordnete Senke ein, so daß auch das Schneidblatt 21a aufgestellt wird. Wird jedoch das Außenrohr 9a entgegen der Richtung x zurückgezogen, so nehmen die Bolzen 31 in der Führungsausnehmung 30 die Schneidblätter 21a bzw. 22a wieder mit und öffnen die Schneidzangeneinrichtung 20a.

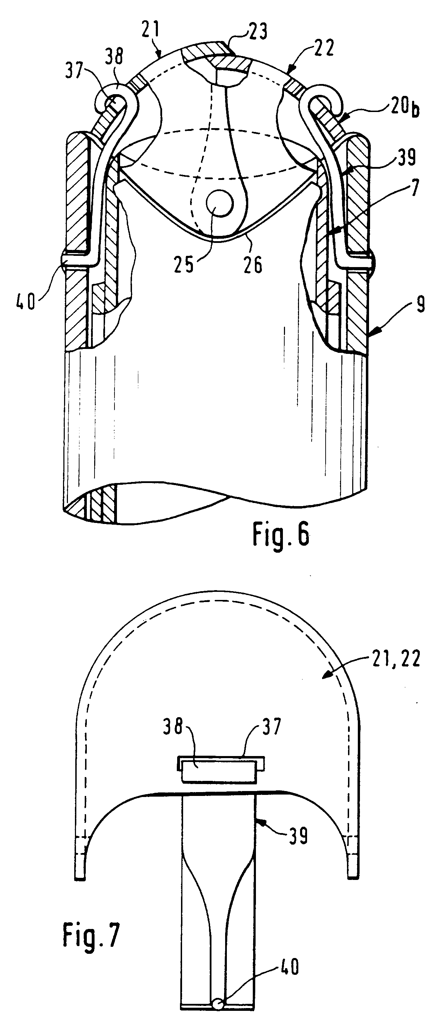

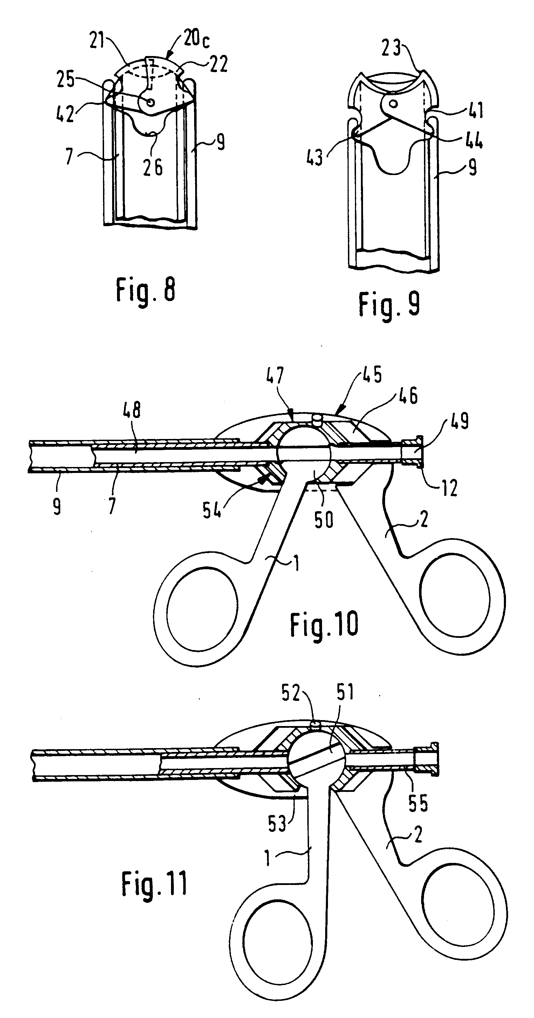

Bei einer Schneideinrichtung 20b gemäß den Figuren 6 und 7 ist ebenfalls ein Innenrohr 7 in ein Außenrohr 9 eingesetzt. Die beiden Schneidblätter 21 und 22 besitzen wiederum den gemeinsamen Drehpunkt 25 am Innenrohr 7. Im gezeigten Ausführungsbeispiel überlappen sich die Schneiden 23.

Jedes Schneidblatt 21 bzw. 22 besitzt eine Ausnehmung 37, wie sie insbesondere in Fig. 7 deutlich gezeigt ist. In diese Ausnehmung 37 greift ein Haken 38 einer Befestigungslasche 39 ein. Andernends des Hakens 38 ist die Befestigungslasche 39 mit dem Außenrohr 9 fest verbunden, wobei sie mit einem entsprechenden Bolzenstück 40 in dem Außenrohr 9 sitzt.

Hier kann dieses Bolzenstück 40 mit dem Außenrohr 9 beispielsweise verschweißt sein.

Deutlich erkennbar ist auch die Senke 26 im Außenrohr 9, welche das ordnungsgemäße Schließen der Schneidblätter 21 und 22 bewirkt. Wird somit das Außenrohr 9 in Richtung x verschoben, so folgen dem Außenrohr 9 auch die Befestigungslaschen 39. Diese nehmen wiederum die Schneidblätter 21 und 22 mit. Dabei fahren diese Schneidblätter 21 und 22 in die Senke 26 ein, so daß ein Schließen bewirkt wird, wobei die Schneiden 23 sich überlappen. Damit ist beispielsweise ein Gewebestück abgeschnitten und kann nun im Innenrohr weitertransportiert werden.

Wird das Außenrohr entgegen der Richtung x verschoben, so nehmen die Befestigungslaschen 39 die jeweiligen Schneidblätter 21 bzw. 22 mit, so daß diese um den Drehpunkt 25 drehen und sich öffnen.

Ein weiteres, sehr einfaches Ausführungsbeispiel einer Schneideinrichtung 20c ist in den Figuren 8 und 9 gezeigt. Auch hier umfängt das Außenrohr 9 das Innenrohr 7, wobei die Öffnungs- und Schließbewegung der Schneideinrichtung 20c von dem Außenrohr 9 bewirkt wird. Die Schneidblätter 21 und 22 drehen wiederum um den Drehpunkt 25, welcher sie am Innenrohr 7 festlegt.

Erfindungsgemäß weist jedoch jedes Schneidblatt 21 bzw. 22 eine Eingriffsmulde 41 auf, in welche eine entsprechend geformte Rastnase 42 des Außenrohres 9 eingreift. Ferner besitzt auch jedes Schneidblatt 21 bzw. 22 neben der Eingriffsmulde 41 eine entsprechende Rastnase 43, die wiederum in eine Eingriffsmulde 44 im Außenrohr 9 eingreift.

Durch dieses Zusammenspiel von Eingriffsmulde 41 und Rastnase 42 sowie Rastnase 43 und Eingriffsmulde 44 erfolgt beim Hin- und Herschieben des Außenrohres 9 ein Öffnen und Schließen der Schneideinrichtung 20c.

In den Figuren 10 und 11 ist ein weiteres Ausführungsbeispiel der Betätigungseinrichtung einer Probenexzisionszange gezeigt. Das Verschieben von Innenrohr 7 zu Außenrohr 9 erfolgt durch den Zangenschenkel 1. Das Außenrohr 9 liegt in einem Gehäuse 45 fest, welches dem anderen Zangenschenkel 2 angeformt ist und eine Mulde 46 zur Aufnahme einer Ventileinrichtung 47 aufweist. Mit dieser Ventileinrichtung 47 kann eine Verbindung zwischen einer Axialbohrung 48 im Innenrohr 7 und dem Anschlußnippel 12 bzw. dessen Innenbohrung 49 hergestellt werden. Wie oben erwähnt, wird über den Anschlußnippel 12 die Gewebeprobe durch die Ansaugöffnung 33 und die Axialbohrung 48 angesaugt.

Zum Öffnen und Schließen der Ventileinrichtung 47 besitzt der Zangenschenkel 1 einen Drehkopf 50 mit einer Querrinne bzw. -bohrung 51. Die Lagerung des Schenkels 1 erfolgt über einen Lagerstift 52 im Gehäuse 45. Andererseits dieses Lagerstiftes 52 durchfährt der Zangenschenkel 1 eine entsprechende Längsöffnung 53 im Gehäuse 45.

Der Drehkopf 50 lagert drehbar in einem Einsatz 54, welcher bei der Bewegung des Zangenschenkels 1 in der Mulde 46 gleiten kann. Dieser Einsatz 54 ist einerseits mit dem Innenrohr 7 und andererseits mit einem Rohrstück 55 verbunden, welches wiederum in der Innenbohrung 49 des Anschlußnippels 12 gleitet.

In der in Fig. 10 gezeigten Öffnungslage der Ventileinrichtung 47 verbindet die Querbohrung 51 die Axialbohrung 48 mit der Innenbohrung 49. Dabei ist die Schneideinrichtung 20 geöffnet, so daß Gewebe in die Ansaugöffnung 33 eingesaugt werden kann.

Wird nun der Zangenschenkel 1 in Richtung auf den Zangenschenkel 2 hin bewegt, so gleitet das Innenrohr 7 in dem Außenrohr 9 und es kommt zu einem Schließen der Schneidblätter 21 und 22, da diese in die entsprechende Senke 26 der Schneideinrichtung 20 eingleiten. Gleichzeitig wird die Ventileinrichtung 47 in der in Fig. 11 gezeigten Schließstellung bewegt, so daß es nicht mehr zu einem weiteren Ansaugen der Gewebeproben kommt. Nach dem Abschneiden kann nun die Ventileinrichtung 47 wieder geöffnet und die Gewebeprobe abgesaugt werden. Wird nun beispielsweise die Zange bzw. die Schneidzangeneinrichtung 20 im menschlichen Körper an eine andere Stelle bewegt, so wird die Schneidzangeneinrichtung 20 geschlossen, so daß die Ventileinrichtung 47 unterbrochen ist. Dabei wird ein dauerndes Ansaugen von unerwünschten Teilchen verhindert.

Die vorliegende erfindungsgemäße Probenexzisionszange soll nicht nur zum Entnehmen von Gewebeproben dienen, sondern auch beispielsweise zum Entfernen eines Teils einer Nervenbahn, eines Venenabschnittes oder eines dergleichen länglichen Körperelementes. Hierzu wird beispielsweise eine Nervenbahn in die Ansaugöffnung des Innenrohres 7 bzw. 7a eingeführt und dann Innenrohr 7 bzw. 7a und Außenrohr 9 bzw. 9a entlang der Nervenbahn über diese geschoben.

Um den Nerv jedoch nach dem Einführen festhalten zu können, ist sowohl im Innenrohr 7 bzw. 7a als auch im Außenrohr 9 bzw. 9a ein Kerbschnitt 34 bzw. 35 vorgesehen. Durch diese Kerbschnitte 34 bzw. 35 ist der Zugriff zum Inneren des Innenrohres 7 bzw. 7a gewährleistet.

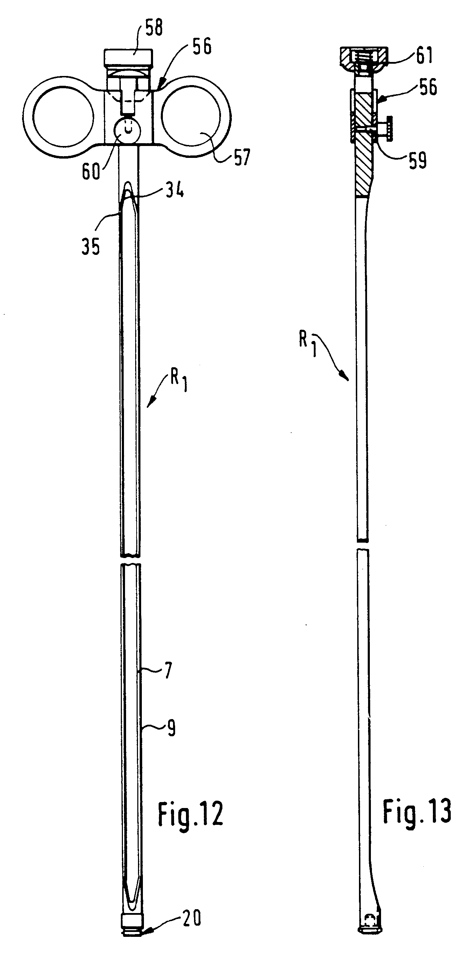

In den Figuren 12 und 13 ist ein vereinfachtes Ausführungsbeispiel einer derartigen Zange R1 gezeigt. Hierbei gleitet das Außenrohr 9 über das Innenrohr 7 hinweg und betätigt die Schneidzangeneinrichtung 20, wie dies oben in mehreren Ausführungsbeispielen beschrieben ist. Ferner ist der entsprechende Kerbschnitt 34 bzw. 35 erkennbar.

Am oberen Ende befindet sich ein Griffstück 56 mit zwei Griffösen 57. Das Innenrohr 7 ist mit diesem Griffstück 56 fest verbunden, während das Außenrohr 9 durch Druck auf einen Schraubkopf 58 betätigt werden kann. Dabei wird das Außenrohr 9 an einem Stift 59 geführt, welcher ein Langloch 60 im Außenrohr 9 durchsetzt.

Es ist vorgesehen, daß der Schraubkopf 58 mit einem Gewinde 61 in eine entsprechende Bohrung im Innenrohr 7 eingreift, so daß ein Verschieben des Außenrohres 9 auch durch dieses Schrauben erfolgen kann. Trifft dabei der Schraubkopf 58 auf einen nicht näher gezeigten Anschlag, so bedeutet dies, daß die Schneidblätter 21 und 22 der Schneideinrichtung 20 geschlossen sind.

## Patentansprüche

1. Medizinische Vorrichtung mit einer Schneidzangeneinrichtung zum Entnehmen von Gewebeproben, Entfernen von langgestreckten Körperelementen, wie Nerven- oder Venenabschnitten od. dgl., wobei ein Innenrohr (7) in ein Außenrohr (9) eingesetzt und ein Rohr (7 oder 9) axial gegen das andere Rohr (7 oder 9) verschiebbar ist und wobei ein Rohr (7 oder 9) die Schneidzangeneinrichtung (20) besitzt, deren Schließbewegung von dem jeweils anderen Rohr (7 oder 9) abhängig ist, wobei die Schneidzangeneinrichtung (20) aus zwei Schneidblättern (21, 22) besteht, welche Schneiden (23) aufweisen, und sich von jeweils einem gemeinsamen Drehpunkt (25) mit einem Rohr (7 oder 9) zu einem gegenüberliegenden, gemeinsamen Drehpunkt mit dem Rohr (7 und 9) erstrecken,
dadurch gekennzeichnet,
daß das jeweils andere Rohr (7 oder 9) stirnwärtig offene Senken (26) in der Stirnkante (27) aufweist, in welche beim Schließen der Schneidzangeneinrichtung (20) durch das axiale Verschieben der Rohre (7, 9) die Drehpunkte (25) einfahren, wobei Ränder der Senken (26) an den Schneiden (23) gegenüberliegenden Außenrändern der Schneidblätter (21, 22) entlanggleiten und die Schneidzangeneinrichtung schließen.

2. Vorrichtung nach Anspruch 1, dadurch gekennzeichnet, daß jeweils einem Drehpunkt (25) eine in die Senke (26a) einfahrende Zunge (28) des einen Schneidblattes (22a) zugeordnet ist, während das andere Schneidblatt (21a) durch eine Schwinge (29) eine Führungsausnehmung (30) ausbildet, welche mit einem Bolzen (31) am anderen Rohr (9a) in Eingriff steht.

3. Vorrichtung nach Anspruch 1, dadurch gekennzeichnet, daß die Schneidblätter (21,22) über Befestigungslaschen (39) mit dem Rohr (7 oder 9) verbunden sind.

4. Vorrichtung nach Anspruch 3, dadurch gekennzeichnet, daß die Befestigungslaschen (39) einen Haken (38) besitzen, mit dem sie in entsprechende Ausnehmungen (37) der Schneidblätter (21,22) eingreifen, während sie andernends über Bolzenstücke (40) od. dgl. mit dem Rohr (7 oder 9) verbunden sind.

5. Vorrichtung nach Anspruch 1, dadurch gekennzeichnet, daß die Schneidblätter (21,22) Eingriffsmulden (41) und Rastnasen (43) aufweisen, welche in entsprechende Eingriffsmulden (44) im Außenrohr (9) eingreifen, während Rastnasen (42) am Außenrohr (9) in Eingriff mit den Eingriffsmulden (41) stehen.

6. Vorrichtung nach wenigstens einem der Ansprüche 1 bis 5, dadurch gekennzeichnet, daß sowohl Außenrohr (9) wie auch Innenrohr (7) in ihrer Mantelfläche einen Kerbschnitt (34,35) aufweisen.

7. Vorrichtung nach wenigstens einem der Ansprüche 1 bis 6, dadurch gekennzeichnet, daß das Innenrohr (7) fest mit einem Griffstück (56) verbunden ist, während sich das Außenrohr (9) gegen einen axial bewegbaren Schraubkopf (58) abstützt.

8. Vorrichtung nach Anspruch 7, dadurch gekennzeichnet, daß der Schraubkopf (58) mit einem Gewindeabschnitt (61) in das Innenrohr (7) eingreift.

9. Vorrichtung nach Anspruch 7 oder 8, dadurch gekennzeichnet, daß das Außenrohr (9) über ein Langloch (60) geführt ist, welches ein Stift (59) durchsetzt, wobei der Stift (59) am Griffstück (56) festliegt.

10. Vorrichtung nach wenigstens einem der Ansprüche 1 bis 9, dadurch gekennzeichnet, daß Außen- und Innenrohr (7 bzw. 9) aus flexiblem Material bestehen.

11. Vorrichtung nach wenigstens einem der Ansprüche 1 bis 10, dadurch gekennzeichnet, daß das Außenrohr (9) über Gelenklaschen (10) mit einer einem Zangenschenkel (1) angeformten Hülse (8) verbunden ist, wobei diese das Innenrohr (7) spielfrei umgibt.

12. Vorrichtung nach wenigstens einem der Ansprüche 1 bis 11, dadurch gekennzeichnet, daß das Innenrohr (7) eine einem Schenkel (2) angeformte Hülse (6) durchsetzt und andererseits einen Anschlußnippel (12) für eine Leitung (13) aufweist.

13. Vorrichtung nach Anspruch 12, dadurch gekennzeichnet, daß das Außenrohr (9) fest mit einem Gehäuse (45) verbunden ist, in welchem sich eine Ventileinrichtung (47) befindet, die eine Verbindung zwischen einer Axialbohrung (48) im Innenrohr (7) und der Leitung (13) herstellt oder unterbricht.

14. Vorrichtung nach Anspruch 13, dadurch gekennzeichnet, daß das Innenrohr (7) mit einem Einsatz (54) verbunden ist, welcher andererseits mit einem Rohrstück (55) in den Anschlußnippel (12) eingreift.

15. Vorrichtung nach Anspruch 14, dadurch gekennzeichnet, daß der Einsatz (54) verschiebbar in einer Mulde (46) des Gehäuses (45) lagert.

16. Vorrichtung nach Anspruch 15, dadurch gekennzeichnet, daß im Einsatz (54) ein Drehkopf (50) drehbar angeordnet ist, welcher eine Querbohrung (51) zum Verbinden von Axialbohrung (48) und Leitung (13) besitzt.

17. Vorrichtung nach Anspruch 16, dadurch gekennzeichnet, daß der Drehkopf (50) mit einem Lagerstift (52) in das Gehäuse (45) eingreift.

18. Vorrichtung nach Anspruch 17, dadurch gekennzeichnet, daß andererseits des Lagerstiftes (52) an den Drehkopf (50) der eine Zangenschenkel (1) angeformt ist, welcher eine entsprechende Ausnehmung (53) im Gehäuse (45) durchsetzt.

19. Vorrichtung nach wenigstens einem der Ansprüche 12 bis 18, dadurch gekennzeichnet, daß die Leitung (13) mit einer Vakuumpumpe (18) in Verbindung steht.

20. Vorrichtung nach Anspruch 19, dadurch gekennzeichnet, daß in die Leitung (13) vor der Vakuumpumpe (18) eine Auffangvorrichtung (14) für die entnommenen Gewebeproben eingeschaltet ist.

## Claims

1. Medical device with a cutting plier arrangement for the extraction of tissue samples, removal of elongate body elements such as portions of nerves or veins or the like, wherein an internal tube (7) is inserted into an external tube (9) and one tube (7 or 9) is axially displaceable against the other tube (7 or 9) and wherein one tube (7 or 9) has the cutting plier arrangement (20) of which the closure movement is dependent on the other respective tube (7 or 9), the cutting plier arrangement (20) consisting of two cutting blades (21, 22) which have cutting edges (23) and extend in each case from a common pivot pin (25) with a tube (7 or 9) to an opposing common pivot pin with the tube (7 and 9), characterised in that the other respective tube (7 or 9) has bays (26), which are open towards the end face, in the end face edge (27) which the pivot pins (25) enter during the closure of the cutting plier arrangement (20) owing to the axial displacement of the tubes (7, 9), the borders of the bays (26) sliding along external borders of the cutting blades (21, 22) opposing the cutting edges (23) and closing the cutting plier arrangement.

2. Device according to Claim 1, characterised in that a tongue (28) entering the bay (26a) of one cutting blade (22a) is allocated to a respective pivot pin (25) while the other cutting blade (21a), owing to a rocker (29), forms a guide recess (30) which engages with a stud (31) on the other tube (9a).

3. Device according to Claim 1, characterised in that the cutting blades (21, 22) are connected to the tube (7 or 9) via fastening straps (39).

4. Device according to Claim 3, characterised in that the fastening straps (39) have a hook (38) with which they engage in corresponding recesses (37) in the cutting blades (21, 22) whereas they are connected to the tube (7 or 9) at the other end via stud members (40) or the like.

5. Device according to Claim 1, characterised in that the cutting blades (21, 22) have indentations (41) and catch projections (43) which engage in corresponding indentations (41) in the external tube (9) whereas catch projections (42) engage with the indentations (41) on the external tube (9).

6. Device according to at least one of Claims 1 to 5, characterised in that the external tube (9) as well as the internal tube (7) have a notch (34, 35) in their surface.

7. Device according to at least one of Claims 1 to 6, characterised in that the internal tube (7) is rigidly connected to a handle (56) whereas the external tube (9) rests against an axially movable screw head (58).

8. Device according to Claim 7, characterised in that the screw head (58) engages with a threaded portion (61) in the internal tube (7).

9. Device according to Claim 7 or 8, characterised in that the external tube (9) is guided via a slot (60) which is penetrated by a pin (59), the pin (59) resting on the handle (56).

10. Device according to at least one of Claims 1 to 9, characterised in that external tube and internal tube (7 and 9) consist of flexible material.

11. Device according to at least one of Claims 1 to 10, characterised in that the external tube (9) is connected via links (10) to a sleeve (8) shaped on one plier arm (1), the sleeve (8) surrounding the internal tube (7) without clearance.

12. Device according to at least one of Claims 1 to 11, characterised in that the internal tube (7) penetrates a sleeve (6) shaped on one arm (2) and, on the other hand, has a connecting nipple (12) for a pipe (13).

13. Device according to Claim 12, characterised in that the external tube (9) is rigidly connected to a casing (45) in which there is located a valve arrangement (47) which produces or interrupts a connection between an axial bore (48) in the internal tube (7) and the pipe (13).

14. Device according to Claim 13, characterised in that the internal tube (7) is connected to an insert (54) which engages, on the other hand, with a length of tube (55) in the connecting nipple (12).

15. Device according to Claim 14, characterised in that the insert (54) is displaceably supported in an indentation (46) in the casing (45).

16. Device according to Claim 15, characterised in that a rotating head (50) having a transverse bore (51) for the connection of axial bore (48) and pipe (13) is rotatably arranged in the insert (54).

17. Device according to Claim 16, characterised in that the rotating head (50) engages, with a bearing pin (54) in the casing (45).

18. Device according to Claim 17, characterised in that one plier arm (1), which penetrates a corresponding recess (53) in the casing (45), is shaped on the rotating head (50) on the other side of the bearing pin (52).

19. Device according to at least one of Claims 12 to 18, characterised in that the pipe (13) is connected to a vacuum pump (18).

20. Device according to Claim 19, characterised in that a receiver (14) for the extracted tissue samples is connected into the pipe (13) before the vacuum pump (18).

## Revendications

1. Appareil médical équipé d'un outil à pince coupante pour prélever des échantillons de tissu, retirer des éléments organiques allongés, tels que des parties de nerfs, de vaisseaux etc, comportant un tube intérieur (7) logé dans un tube externe (9) avec possibilité de coulissement relatif, l'un des tubes (7 ou 9) portant l'outil à pince coupante (20) dont la fermeture dépend de l'autre tube (7 ou 9) et qui est constitué de deux lames coupantes (21, 22) présentant des tranchants (23) et reliant chacune au point commun de rotation (25) porté par un tube (7 ou 9) à un autre point commun de rotation, situé à l'opposé et porté par le même tube (7 et 9), caractérisé en ce que l'autre tube (7 ou 9) présente dans son bord frontal (27) des découpes (26) ouvertes vers l'extérieur et dans lesquelles, lors de la fermeture de l'outil à pince coupante (20) pour déplacement relatif axial des tubes (7, 9), pénètrent les points de rotation (25), de sorte que les bords des découpes (26) glissent le long des bords externes des lames de coupe (21, 22) opposés aux tranchants (23) en provoquant la fermeture de l'outil à lames coupantes.

2. Dispositif selon la revendication 1, caractérisé en ce qu'à chaque point commun de rotation (25) est associée une languette (28) appartenant à une des lames de coupe (22a) et pénétrant dans la découpe (26a) tandis que l'autre lame de coupe (21a) par l'intermédiaire d'une partie courbe (29) en forme de coulisse dessine une ouverture de guidage (30), en prise avec un pivot (31) porté par l'autre tube (9a).

3. Dispositif selon la revendication 1, caractérisé en ce que les lames de coupe (21, 22) sont reliées au tube (7 ou 9) par des pattes de fixation (39).

4. Dispositif selon la revendication 3, caractérisé en ce que les pattes de fixation (39) comportent un crochet (38) par lequel elles sont en prise avec des évidements correspondants (37) des lames de coupe (21, 22), tout en étant reliées par ailleurs au tube (7 ou 9) par des pivots (40) ou organes analogues.

5. Dispositif selon la revendication 1, caractérisé en ce que les lames de coupe (21, 22) comportent d'une part des parties en creux (41) et des becs d'arrêt (43) qui viennent en prise dans des cavités (44) corespondantes, dans le tube externe (9) tandis que les becs d'arrêt (42) sont en prise dans les cavités (41) du tube externe (9).

6. Dispositif selon au moins une des revendications 1 à 5, caractérisé en ce que le tube externe (9) comme le tube interne (7) portent chacun, sur leur paroi externe, une entaille (34, 35).

7. Dispositif selon au moins une des revendications 1 à 6, caractérisé en ce que le tube interne (7) est solidaire d'une pièce de saisie manuelle (56) tandis que le tube externe (9) est en appui sur une tête de vis (58) mobile axialement.

8. Dispositif selon la revendication 7, caractérisé en ce que la tête de vis (58) est en prise par une partie filetée (61) dans le tube interne (7).

9. Dispositif selon la revendication 7 ou 8, caractérisé en ce que le tube externe (9) est guidé par une boutonnière (60) traversée par une tige (59) qui se trouve donc maintenue le long de la pièce de saisie (56).

10. Dispositif selon au moins une des revendications 1 à 9, caractérisé en ce que les tubes externe et interne (7 et 9) sont réalisés en un matériau flexible.

11. Dispositif selon au moins une des revendications 1 à 10, caractérisé en ce que le tube externe (9) est relié par des pattes d'articulation (10) à une douille (8) formée à l'extrémité d'une branche de pince (1) et entourant sans jeu le tube interne (7).

12. Dispositif selon au moins une des revendications 1 à 11, caractérisé en ce que le tube interne (7) traverse une douille (6) formée d'une branche (2) et comporte par ailleurs un ajutage (12) de raccordement à une canalisation (13).

13. Dispositif selon la revendication 12, caractérisé en ce que le tube externe (9) est solidaire d'un boîtier (45) dans lequel est logé un dispositif à soupape établissant ou interrompant la liaison entre un alésage axial (48) du tube interne (7) et la canalisation (13).

14. Dispositif selon la revendication 13, caractérisé en ce que le tube interne (7) est raccordé à un embout (54) lui-même engagé par une tubulure (55) dans l'ajutage de raccordement (12).

15. Dispositif selon la revendication 14, caractérisé en ce que l'embout (54) peut coulisser dans une chambre (46) du boîtier (45).

16. Dispositif selon la revendication 15, caractérisé en ce que dans l'embout (54) est montée une tête tournante (50) percée d'un alésage transversal (51) permettant de mettre en liaison l'alésage axial (48) et la canalisation (13).

17. Dispositif selon la revendication 16, caractérisé en ce que la tête tournante (50) est en prise dans le boîtier (45) par l'intermédiaire d'une tige de positionnement (52).

18. Dispositif selon la revendication 17, caractérisé en ce qu'à l'opposé de la tige de positionnement (52), la tête tournante (50) porte une branche de pince (1) traversant une ouverture correspondante (53) du boîtier (45).

19. Dispositif selon au moins une des revendications 12 à 18, caractérisé en ce que la canalisation (13) est reliée à une pompe à vide (18).

20. Dispositif selon la revendication 19, caractérisé en ce qu'un dispositif de réception (14) des échantillons de tissu prélevés est monté sur la canalisation (13), en amont de la pompe à vide (18).
